Europäisches Patentamt

**European Patent Office**

Office européen des brevets

(11) Publication number: **0 125 122**
A2

(12) **EUROPEAN PATENT APPLICATION**

(21) Application number: **84303030.5**

(22) Date of filing: **04.05.84**

(51) Int. Cl.³: **A 61 M 5/14**

(30) Priority: **10.05.83 US 493187**

(43) Date of publication of application:
**14.11.84 Bulletin 84/46**

(84) Designated Contracting States:
**AT BE CH DE FR GB IT LI LU NL SE**

(71) Applicant: **ANATROS CORPORATION**
**1922 Junction Avenue**
**San Jose California 95131(US)**

(72) Inventor: **Danby, Hal C.**
**3863 Ross Road**
**Palo Alto California 94303(US)**

(72) Inventor: **Ritson, Carl**
**1537 Montellano Drive**
**San Jose California 95120(US)**

(72) Inventor: **Redmond, William F.**
**1872 Jonston Avenue**
**San Jose California 95125(US)**

(74) Representative: **Ellis, John Clifford Holgate et al,**
**MEWBURN ELLIS & CO. 2/3 Cursitor Street**
**London EC4A 1BQ(GB)**

(54) Parenteral solution delivery system.

(57) A parenteral solution delivery control system includes tubing (4) leading from a liquid source to a drop former (16) (which may be of adult or pediatric configuration) whereby drops fall through the drip chamber (8) onto the surface of the liquid therein. An outlet (10) communicates through control valve (12) to tubing (14) which leads to a patient. The system includes an air-in-line detector opposite tubing (4); a drop former monitor; and a drop detector and liquid level monitor across chamber (8). In each of these detectors and monitors a light emitter (24) and detector (34) are mounted on parallel axes, and a pair of deflecting, focusing prisms (20, 40) are positioned to direct the light through the desired zone.

./...

Fig_1

Fig_3

## Title of the Invention

PARENTERAL SOLUTION DELIVERY SYSTEM

## Field of the Invention

This invention relates to an apparatus for delivering parenteral solutions to patients. In particular, this invention is directed to an improved delivery apparatus having greatly improved safety features.

## Background of the Invention

### DESCRIPTION OF THE PRIOR ART

Infusion delivery systems for delivering liquid to a patient from more than one solution source have been previously known. The most common systems use gravity flow and manually adjustable tubing clamps or pinch valves. They may employ a variety of valves and junctions to control flow at the desired rate and sequence. Examples of such systems are described in U.S. patents 3,886,937; 4,034,754; 4,114,617; 4,219,022; 4,223,695; 4,236,515; 4,237,879; 4,237,880; 4,250,879; 4,252,116; 4,256,104; 4,256,105; and 4,258,712. Dual delivery systems relying on electronic flow control means are described in U.S. Patent 4,094,318, for example.

Automatic flow control systems relying on a drop counter which measures the frequency of drop fall through a drip chamber have been previously known. In general, a light beam from a lamp to a light detector is positioned so that it is interrupted by drops falling through a drip chamber. The frequency of the breaking of the light beam and/or the time lapse between drops breaking the light beam are directly proportional to the flow rate and are used to determine adjustments to be made to a flow control valve to change flow to the desired rate. Examples of systems comprising drop counters and control systems responsive thereto are described in U.S. patents 3,163,179; 3,601,124; 3,886,937; 4,038,982; 4,314,567.

## SUMMARY AND OBJECTS OF THE INVENTION

It is an object of this invention to provide an improved parenteral solution delivery system having an improved air-in-line detector, drop detector, drop former monitor, or liquid level monitor.

It is a further object of this invention to provide a light source and light detector system including improved optics which has greater accuracy and reliability than previously used components.

The parenteral solution delivery control system of this invention comprises a receptor for a drip chamber having a drop zone through which drops fall when combined with a drip chamber. A drop detector is positioned to pass a light beam through the drop zone. The light detector comprises a light source and a light detector on parallel axes. An emitter prism is positioned adjacent a light source for receiving light from the light source, deflecting it 90° and passing it through a beam forming lens to emit a light beam passing through the drop zone. A detector receives said light beam after it passes through the drop zone, passing it through a collector lens, deflecting it 90° and directing it to the light detector.

The air-in-line detector system of this invention comprises a single light source and a single light detector for detecting the presence of air in tubing. The system functions with both transparent and opaque liquids. The light source is positioned to pass light through a tubing zone. A mask has an opening positioned to direct beam light (permit passage of beam light) only onto the tubing surface intermediate the central axis and the edge of the tubing. A detector mask has an opening positioned to permit passage of unfocused light emitted when the tubing is filled with air, but to block passage of focused beam light which is produced when the tubing is filled with a transparent liquid. With this system, light passes from the light source to the light detector only when the tubing is filled with air.

The drop former monitor in the parenteral solution delivery control system of this invention includes an optical system which distinguishes between adult and pediatric drop formers. A difference in transparency or silhouettes is used to block a light beam when the delivery system is in one or the other of the pediatric or adult modes but does not block the light beam in the alternate mode. A light source means is positioned to direct a beam of light through a path which can be blocked by the drop former when the blocking mode is in place. A light detector is positioned in the path of the unblocked light beam.

The drip chamber level monitor in the parenteral solution delivery control system of this invention comprises a level detector positioned with respect to the normal liquid level in a drop detector to signal when the liquid level therein exceeds a predetermined level. It provides this function for both transparent and opaque liquids. The detector system includes a single light source and a single light detector which detects the presence of air in the above-surface zone of a drip chamber, and conversely can detect the presence of liquid in the above-surface zone when the liquid level exceeds the normal or intended surface level. The light source is positioned to pass light through a drip chamber and is associated with an emitter mask having an opening positioned to direct beam light through a drip chamber intermediate the drip chamber axis and the outer edge of the drip chamber. A detector mask is positioned to permit passage of unfocused beam light when the drip chamber is filled with air but is aligned to block passage of focused beam light which is produced when the drip chamber is filled with a transparent liquid. Failure of light to reach the detector indicates that liquid (transparent or opaque) has risen into the normally above-surface zone.

Brief Description of the Drawings

Figure 1 is a frontal view of one form of parenteral solution delivery control system according to this invention.

Figure 2 is a cross-sectional representation of the parenteral solution delivery control system taken along the line 2-2 in Figure 1.

Figure 3 is a schematic representation of the optic systems in the parenteral delivery control system.

Figure 4 is a cross-sectional representation of the parenteral solution delivery control system showing details of the air-in-line detector and is taken along the line 4-4 in Figure 1.

Figure 5 is a cross-sectional representation of the parenteral solution delivery control system showing the drop former monitor (adult configuration) and is taken along the line 5-5 in Figure 1.

Figure 6 is a partial side-sectional view of a drip chamber with a pediatric size drop former.

Figure 7 is a cross-sectional representation of the parenteral solution delivery control system showing the drop former monitor (pediatric size) and is taken along the line 5-5 in Figure 1.

Figure 8 is a cross-sectional representation of the parenteral solution delivery control system showing the drop detector and is taken along the line 8-8 in Figure 1.

Figure 9 is a cross-sectional representation of the parenteral solution delivery control system showing the liquid level monitor and is taken along the line 9-9 in Figure 1.

Detailed Description of the Invention

An improved parenteral solution delivery control system is described in commonly assigned, copending application Serial No. 480,527, filed March 30, 1983, and an improved precision valve assembly suitable for use in that system is described in commonly assigned, copending application Serial No. 431,312 filed September 30, 1982. The entire contents of both applications are hereby incorporated by reference in their entirety. These systems provide a predetermined volume of parenteral solution to a patient at a precisely controlled flow rate. This invention is directed to a control system which provides safety features, increasing the reliability and reducing the risks associated with parenteral solution delivery systems.

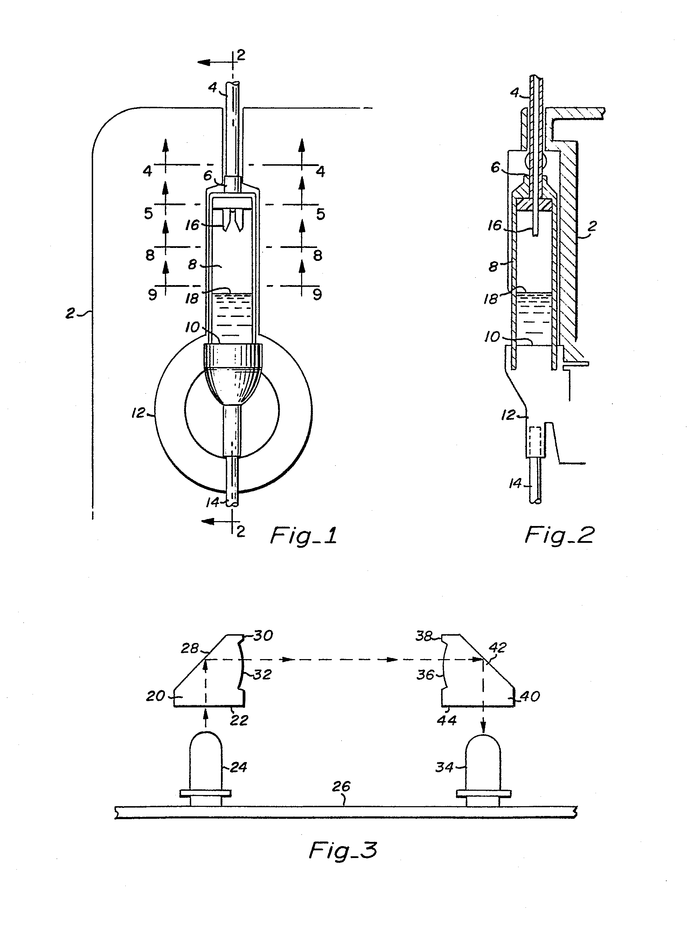

Figure 1 shows a frontal view of one form of the parenteral solution delivery control system of the invention, and Figure 2 is a cross-sectional representation taken along the line 2-2 in Figure 1. The control housing 2 (shown schematically) is designed to receive the delivery system components. Tubing 4 leads from a liquid source (not shown) to the inlet 6 of drip chamber 8. The drip chamber outlet 10 leads to the control valve 12. Tubing 14 leads from the control valve to the patient. The liquid entering the drip chamber passes through a drop former 16 which effects formation of evenly sized droplets. The drop former 16, as shown, has the adult configuration. The normal liquid level surface 18 is sufficiently high to permit air bubbles entrained by falling drops to separate from the liquid.

Figure 3 is a schematic representation of the optics system used in the control systems of this invention. The emitter prism 20 is positioned with its receptor surface 22 adjacent and in the axial path of the light emitting diode 24 mounted on the board 26. The reflecting surface 28 of the prism 20 forms an angle of 45° with the receptor surface 22 and deflects light in a 90° angle toward the emitting surface 30 as shown by the dotted line. The lens 32 on the emitting surface 30 forms light into a light beam and is oriented in a plane forming an angle of 45° with the reflecting surface 28.

The light detector 34 is mounted axially parallel to the light emitter 24 on the board 26. Light impinging on the collector lens 36 of the receptor surface 38 of the detector prism 40 is directed toward the reflecting surface 42 which forms an angle of 45° with respect thereto. The reflecting surface directs the light through the emitting surface 44 and to the detector adjacent thereto. The prisms 20 and 40 can be identical.

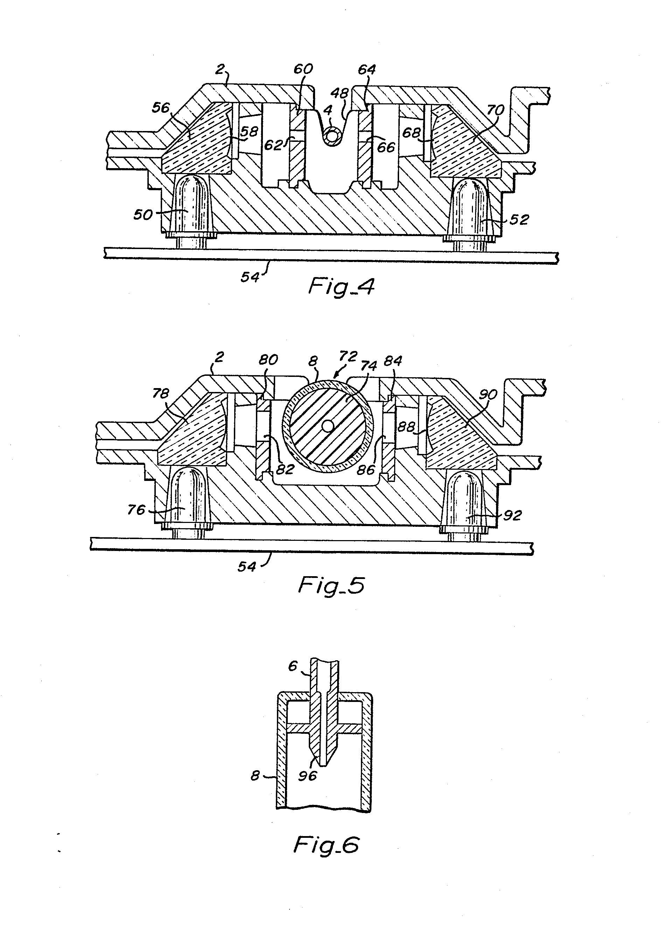

Figure 4 is a cross-sectional representation of the parenteral solution delivery control system of this invention showing the air-in-line detector and is taken along the line 4-4 in Figure 1. The tubing 4 is cradled in a tubing support 48 which connects with the housing 2.

The air-in-line detector comprises a single light source 50 and a single light detector 52 for detecting the presence of air in tubing 4. This functions with both transparent or opaque liquids. The light source assembly comprises a light source 50 which can be a light emitting diode mounted on base plate 54. The emitter prism 56 is positioned in the path of light emitted from the diode 50. The light is deflected 90° and passes through the beam forming lens 58. The lens 58 focuses the deflected light into a beam of parallel light which is directed toward the transparent tubing 4. The emitter mask 60 is made of an opaque material and has a slit or other opening 62 which permits the passage of light only through a precise path with respect to the tubing 4. The tubing 4 is shown in the tubing zone, oriented in a precise position by the support 48. The slit or opening 62 in the emitter mask 60 is positioned to direct the beam light onto the tubing surface intermediate the central tubing axis and the edge of the tubing. The opaque detector mask 64 is positioned in the path of the light passing through the tubing 4 and has an opening or slit 66. The opening or slit 66 is positioned to permit the passage of unfocused beam light, that is, diffused light in the path that light travels when the tubing is filled with air. The mask 64 blocks passage of focused light, that is, light traveling in the path that light travels when the tubing 4 is filled with a transparent liquid. When the tubing 4 is filled with transparent liquid, the tubing and lens functions as a unitary, cylindrical lens, focusing light to a focal point in the manner of a convex lens. When the tubing 4 is filled with an opaque liquid, no beam light reaches the mask opening 66. Beam light reaches the mask opening 66 only when the tubing 4 is filled with air.

The light passing through the mask opening 66 impinges upon the collector lens 68 of the detector prism 70 and is deflected 90° to impinge on the detector 52.

Figure 5 is a cross-sectional representation of the parenteral solution delivery control system showing the drop former monitor and is taken along the lines 5-5 in Figure 1. The housing 2 has a recess 72 which receives the drip chamber 8. The adult size drop former 16 has a cross-section or silhouette 74 which completely blocks the light path with opaque plastic. The light emitting diode 76 mounted on the base plate 54 emits light which is deflected and focused into a beam by prism 78. The opaque emitter mask 80 has an opening 82 which permits the passage of light in a path through the zone occupied by the drop former silhouette 74. As can be seen in this configuration, the light is blocked by the drop former silhouette 74. The opaque detector mask 84 has an opening 86 positioned to receive light which passes through the drop former zone. This light is collected by collector lens 88 in the prism 90, deflected 90°, and directed toward the light detector diode 92.

Figure 6 shows a partial frontal view of a drip chamber 8 having a pediatric size drop former 96. The silhouette of the drop former 96 only blocks a portion of the light path, leaving an open area which permits passage of light through the drop former zone.

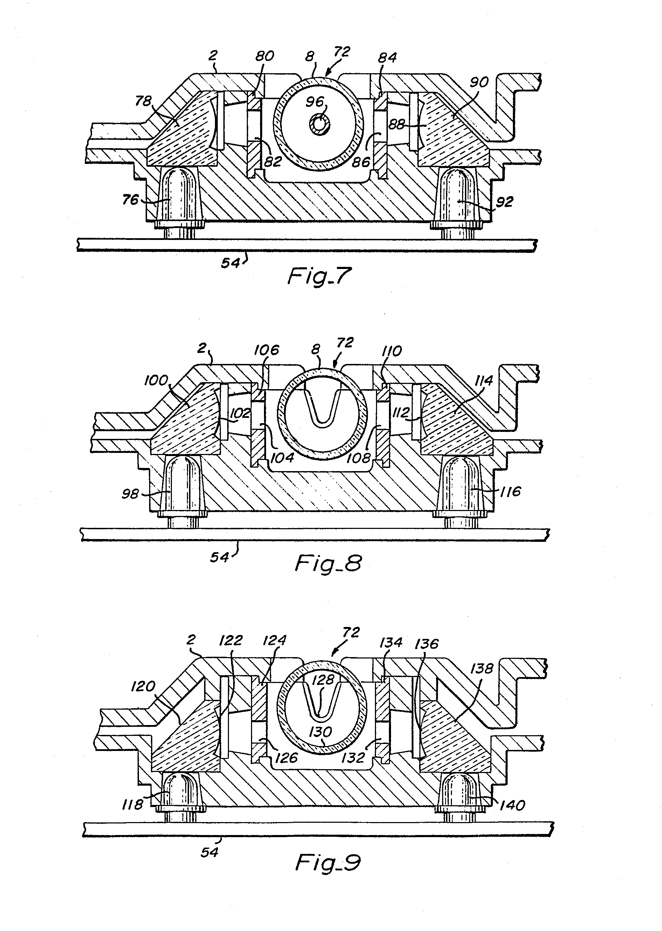

Figure 7 is a cross-sectional representation of the parenteral solution delivery control system taken along the line 5-5 and showing the system with a pediatric drop former 96 in place. As can be seen from this embodiment, light passing through the zone occupied by the drip chamber 8 is not completely blocked by the drop former 96, and a portion of the light passes through the opening 82 in the mask 80 passes to the detector prism 90 where it is reflected to the detector 92. When the pediatric size drop former 96 is in position, the light detector 92 therefore receives light.

Figure 8 is a cross-sectional representation of the parenteral solution delivery control system taken along the line 8-8 in Figure 1 and showing the drop detector system. The housing recess 72 surrounds the drip chamber 8 on three sides, and the drip chamber 8 is positioned in a zone through which light is directed by the optical system. The drops passing through the beam of light interrupt the beam and reflect a portion of the light, causing a corresponding reduction in voltage from a light detector. This signal can be used, by means known per se in the art, to identify the flow rate of liquid passing through the system.

In the configuration shown in Figure 8, the optical system is used to provide a highly reliable, accurate and easily assembled assemblage. Light from the emitter 98 is deflected 90° by prism 100 and is focused into a beam of light by the lens 102. The light passing through the opening 104 in the opaque mask 106 is directed through the zone in drip chamber 8 through which drops pass. The light, after passage through the drop zone, passes through the opening 108 in the opaque mask 110, is focused by the collector lens 112. It is deflected 90° by the prism 114 and directed toward the light detector 116.

Figure 9 is a cross-sectional representation of the parenteral solution delivery control system showing the level control system and is taken along the line 9-9 in Figure 1. This view shows the lower portion of the drip chamber, sectioned just above the normal liquid level 18. This configuration is designed to detect when the liquid level rises to include the zone through which this cross-sectional view is taken.

The light emitting diode 118 emits light which is deflected by prism 120 and focused into a light beam by the lens 122. The opaque mask 124 has an opening 126 which blocks all portions of the light beam except the portion which passes through the drip chamber in an area between the central axis 128 thereof and the inner wall surface 130. The light passing through the zone of air defined by the walls of the drip chamber 8 follows a diffuse path which impinges on the opening 132 of the mask 134. This light is collected by the lens 136, deflected 90° by the prism 138, and directed to the light detector 140.

When the air zone of the light path shown in Figure 8 is occupied by an opaque liquid, no light passes therethrough. When this zone is occupied by a transparent liquid, the walls of the drip chamber 8 and the liquid form a single cylindrical (convex) lens system, and the light beam is focused to impinge on the mask 134 in an opaque area displaced from the opening 132. Therefore, when the zone is filled with transparent liquid, light does not pass through the deflector mask opening 132. When the zone of the light path is filled with either transparent or opaque liquid, no light from the light beam impinges on the light detector 140. However, when the zone is filled with air, light impinges upon the detector 140, and a corresponding detector voltage is generated thereby. Interruption of the voltage signals that the liquid level has risen above the desired level. This can be employed to signal an alarm or terminate operation of the device.

In the control system of this apparatus, the signals generated by the light detectors in each of the components described above is used to regulate and control operation of the system. The intrusion of air into tubing positioned in the air-in-line detector and the occurrence of a liquid level which is above the normal level signal an improper and disfunctional operation of the apparatus, and the signal can be used to signal an alarm and terminate operation of the device.  The drop former monitor can be used to signal to the oeprator which of the drop former sizes are provided and can verify that the appropriate rate settings are used.  In each of the systems, the novel light emitter, detector and prism system combination can be employed to an advantage.

CLAIMS:

1. A parenteral solution delivery control system comprising an air-in-line detector means with a single light source means and a single light detector means for detecting the presence of air in tubing containing either transparent or opaque liquid, said light source means positioned to pass light through a tubing zone, emitter mask means having an opening positioned to direct beam light onto the tubing surface intermediate the central tubing axis and the edge thereof, detector mask means having an opening positioned to permit passage of diffuse beam light but to block passage of focused beam light which is formed when the tubing is filled with a transparent liquid.

2. A parenteral solution delivery control system of Claim 1 comprising a receptor defining a drip chamber zone with a liquid level detector means for signaling when the liquid level in a drip detector exceeds a predetermined level for both transparent and opaque liquids comprising a single light source means and a single light detector means, said light source means positioned to pass beam light through a normally air-filled zone of a drip chamber, emitter mask means having an opening positioned to direct beam through a drip chamber intermediate the central axis and outer edge of a drip chamber detector mask means having an opening positioned to permit passage of diffused beam light when a normally air-filled zone of a drip chamber is filled with air but is positioned to block passage of focused beam light when the normally air-filled zone of a drip chamber is filled with transparent liquid.

3. The parenteral solution delivery control system of Claims 1 and 2 wherein the light source means and light detector means comprise a light source and a light detector and emitter prism means for receiving light from the light source, deflecting it 90°, passing it through a beam forming lens means to emit a light beam passing through the emitter mask opening, and a detector prism means for receiving light passing through a detector mask opening, passing it through a lens, deflecting it 90° and directing it toward the light detector.

4. The parenteral solution delivery control system of Claim 3 wherein the emitter prism means comprises an emitter prism having an emitter light receiving surface positioned for receiving light from the light source, an emitter light emitting surface forming an angle of 90° therewith and including a beam forming lens and an emitter reflecting surface forming 45° angles with the emitter light receiving surface and emitter light emitting surface.

5. The parenteral solution delivery control system of Claim 3 wherein the detector prism means comprises a detector light receiving surface with a collector lens means positioned for receiving the unfocused light beam, a detector light emitting surface which forms an angle of 90° therewith, and a detector reflecting surface forming 45° angles with the detector light receiving surface and the detector light emitting surface.

6. A parenteral solution delivery control system comprising a drip chamber including an opaque drop former means which has a silhouette which blocks light when in a blocking mode corresponding to a pediatric or an adult drop former and which permits light passage when in the respective opposite unblocked mode, a light source means positioned to direct a beam of light through a path blocked by the silhouette when in a blocking mode but not blocked when the alternative unblocked mode, and a light detector in the path of the beam of light to detect when the path is not blocked, signaling that the drop detector is in the unblocked mode.

7. A parenteral solution delivery control system of Claim 6 including a receptor for a drip chamber defining a drop zone through which drops fall from a drop former, a drop detector means positioned to pass a light beam through the drop zone, the drop detector means comprising a light source and a light detector, an emitter prism means positioned for receiving light from the light source, deflecting it 90° and passing it through a beam forming lens means to form a light beam passing through the drop zone, and a detector prism means positioned for receiving said light beam after its passage through the drop zone, deflecting it 90° and directing it to the light detector.

8. A parenteral solution delivery control system comprising a drip chamber zone having a drop zone through which drops fall, a drop detector means positioned to pass a light beam through the drop zone, the drop detector means comprising a light source and a light detector, an emitter prism means for receiving light from the light source, deflecting it 90°, passing it through a beam forming lens means to emit a light beam passing through the drop zone, and a detector prism means for receiving said light beam after its passage through the drop zone, deflecting it 90°, and directing it to the light detector.

9. The parenteral solution delivery system of Claims 7 and 8 wherein the emitter prism means comprises an emitter prism having an emitter light receiving surface for receiving light from a light source, an emitter light emitting surface including a beam forming lens forming an angle of 90° therewith, and an emitter reflecting surface forming 45° angles with the emitter light receiving surface and emitter light emitting surface.

10. The parenteral solution delivery system of Claims 7 and 8 wherein the detector prism means comprises a detector light receiving surface with a collector lens means positioned for receiving said light beam, a detector light emitting surface which forms an angle of 90° therewith, and a detector reflecting surface forming 45° angles with the detector light receiving surface and the detector light emitting surface.

Fig_1

Fig_2

Fig_3

Fig_4

Fig_5

Fig_6

5/5

0125122

Fig. 7

Fig. 8

Fig. 9